# EUROPÄISCHE PATENTSCHRIFT

(11) **EP 3 025 779 B1**
(45) Veröffentlichungstag und Bekanntmachung des Hinweises auf die Patenterteilung: **11.07.2018**
(21) Anmeldenummer: 14003970.2
(22) Anmeldetag: 26.11.2014
(51) Int. Cl.: B01L 3/00

(54) **INKUBATIONSWANNE MIT TESTSTREIFEN**
INCUBATION TRAY WITH TEST STRIP
PLATEAU D'INCUBATION AVEC BANDE-TEST

(43) Veröffentlichungstag der Anmeldung: 01.06.2016
(73) Patentinhaber: Euroimmun Medizinische Labordiagnostika AG, 23560 Lübeck (DE)
(72) Erfinder: Kemsies, Detlef, 23619 Zarpen (DE); Schröder, Dieter, 23617 Stockelsdorf (DE); Nevermann, Stefan, 23560 Lübeck (DE)

(56) Entgegenhaltungen:
- DE-U1-202012 004 404
- US-A- 6 118 582
- US-A1- 2004 013 576
- US-A1- 2007 237 687

## Beschreibung

Die vorliegende Erfindung betrifft eine längliche Inkubationsrinne mit zwei einander gegenüber liegenden Längswänden, einer Vertiefung und einem Boden, bestückbar mit einem länglichen Teststreifen mit einer Rückseite und einer Vorderseite, wobei der Teststreifen mit seiner Rückseite dem Boden der Inkubationsrinne zugewandt ist und auf seiner Vorderseite mit wenigstens einem analytischen Reagenz beschichtet ist, weiter aufweisend ein Befestigungsmittel, ober das die Inkubationsrinne mit wenigstens einer *weiteren* Inkubationsrinne zu einem Verbund von Inkubationsrinnen verbunden werden kann, dadurch gekennzeichnet, dass auf einer der Längswände ein waagerecht nach außen stehender Auflagerahmen angebracht ist, sowie eine längliche Inkubationsrinne mit zwei einander gegenüber liegenden Längswänden, einer Vertiefung und einem Boden, bestückbar mit einem länglichen Teststreifen mit einer Rückseite und einer Vorderseite, wobei der Teststreifen mit seiner Rückseite dem Boden der Inkubationsrinne zugewandt ist und auf seiner Vorderseite mit einem oder mehr als einem analytischen Reagenz beschichtet ist, weiter umfassend wenigstens zwei Halteelemente zur Fixierung des Teststreifens in Bodennähe, dadurch gekennzeichnet, dass die wenigstens zwei Halteelemente jeweils in Form eines aus der Längswand hervorstehenden Vorsprungs ausgebildet sind und eine zur Seite und nach unten offene Aussparung zur Aufnahme des Teststreifens aufweisen.

Auf dem Gebiet der medizinischen Labordiagnostik sind unterschiedliche Testsysteme bekannt, mit denen Patientenproben auf das Vorhandensein spezifischer Antikörper untersucht werden können. Durch solche Nachweise ist es möglich, Rückschlüsse auf das Auftreten von Krankheiten zu ziehen, die zusammen mit derartigen spezifischen Antikörpern auftreten. Die Krankheit kann als Folge der Bildung von Autoantikörpern auftreten oder die Antikörper werden als Reaktion auf die Krankheit gebildet, beispielsweise als Reaktion auf das Eindringen von krankheitserregenden Viren. Beispielhafte Krankheiten umfassen Infektionen, entzündliche Krankheiten wie rheumatoide Krankheiten, Stoffwechselkrankheiten wie Diabetes und neurologische Erkrankungen.

Üblicherweise erfolgt der Nachweis von diagnostisch relevanten Antikörpern im Wege der sogenannten Stufendiagnostik, bei der zunächst ein empfindliches Screening und dann eine spezifische Bestätigung durchgeführt werden. In der Routineserologie werden hierbei für das Screening oftmals ELISA (Enzyme-linked immunosorbent assay) verwendet, während als Bestätigungstest vornehmlich Immuno-Blotstreifen, insbesonderer Westemblot-Streifen, Dot Blot-Streifen oder Linienblot-Streifen eingesetzt werden.

Da der Bedarf an derartigen Testsystemen erheblich ist und die zum Einsatz kommenden Reagenzien oft hochpreisig, schwer zu beschaffen und nur In geringen Mengen verfügbar sind, ist eine Vereinfachung und Optimierung der Abläufe bei der Durchführung des jeweiligen Tests unerlässlich. Insbesondere müssen die verwendeten analytisch-diagnostischen Gerätschaften die möglichst störungsfreie und einfache parallele Durchführung zahlreicher Tests im Hochdurchsatzmaßstab ermöglichen. Jede Vereinfachung von Verfahrens-, Wartungs- oder Reinigungsschritten, jeder Ausschluß von noch so geringen Gefahrenquellen und jede Minimierung der Mengen der einzusetzenden Reagenzien führt für den Anwender zu erheblichen Einsparungen.

Besonderes Augenmerk ist dabei auf die Inkubationsrinnen zu richten, in denen die genannten Teststreifen bei der Durchführung der Tests mit verschiedenen Reagenzien unter verschiedenen Bedingungen inkubiert werden.

Vorrichtungen mit Inkubationsrinnen, die für derartige Testsysteme eingesetzt werden, können allerdings auch auf auf anderen technischen Gebieten eingesetzt werden, bei denen Teststreifen oder andere streifenförmige Träger in einer Flüssigkeit zu inkubieren sind. Beispielsweise werden Teststreifen auch in der chemischen Analytik zur einfachen Bestimmung der Konzentration des pH-Wertes oder von anorganischen Ionen verwandt. Auch bei der Fertigung von Teststreifen o.ä. Trägern kann es notwendig sein, diese einzeln in einer Flüssigkeit zu inkubieren.

Im Stand der Technik sind eine Reihe von Inkubationsrinnen beschrieben, die für verschiedene analytische und diagnostische Tests eingesetzt werden können.

Die DE 20 2012 004 404 U1 offenbart eine Inkubationsrinne in einer Vorrichtung zur Untersuchung einer Patientenprobe, die mit einem länglichen Teststreifen bestückt ist, wobei der Teststreifen mit seiner Rückseite dem Rinnenboden zugewandt in die Inkubationsrinne eingelegt wird. Zur Handhabung wenigstens zweier Inkuabtionsrinnen ist ein Tablett mit einer Vielzahl von Aufnahmen vorgesehen, in die eine Inkubationsrinne und/oder ein Verbund wenigstens zweier Inkubationrinnen einlegbar ist. Zusätzlich zur Fixierung des Verbundes durch das Tablett mit den Aufnahmen kann die Inkubationsrinne an einer Längsseite ein Befestigungselement aufweisen, über das sie mit einer weiteren Inkubationsrinne verbunden werden kann, beispielsweise eine Schnapp- oder Clip-Verbindung.

Die DE 20 2012 004 404 U1 offenbart jedoch nicht, dass wenigstens zwei Inkubationsrinnen im verbundenen Zustand unabhängig von einem Tablett über eine Kombination aus Befestigungselementen an beiden Längsseiten der Inkubationswanne sowie einen Auflagerahmen miteinander verbunden sein können.

So kann ein Verbund der in der DE 20 2012 004 404 U1 beschriebenen Inkubationswannen nicht unabhängig von dem Tablett bewegt werden Die Aufnahmen in dem Tablett sind zur Fixierung und Stabilisierung des Verbundes erforderlich. Ein Halteelement lediglich an einer Längsseite kann bestenfalls zwei kleine Inkubationswannen mit geringem Gewicht zusätzlich fixieren, nicht jedoch einen größeren Verbund, der unabhängig vom Tablett zu bewegen ist.

Zwischen den in der DE 20 2012 004 404 U1 offenbarten Inkubationrinnen liegen Spalten vor, durch die verpritzte Flüssigkeit durch den Verbund auf das Tablett und die Aufnahmen darin fließen kann. Dies bedingt einen hohen Aufwand bei der Reinigung oder die Notwendigkeit, das Tablett mit den Aufnahmen häufig auszutauschen.

Weiterhin offenbart die DE 20 2012 004 404 U1 eine Inkubationsrinne, die mit einem Teststreifen bestückbar ist. Das Aufschwimmen wird in einer Ausführungsform dadurch verhindert, dass der Teststreifen mit Hilfe von Haftkleber am Boden der Inkubationsrinne fixiert wird. Damit ist im Falle eines reversiblen Fixierens jedoch das Risiko verbunden, dass sich der Streifen bei längerer Inkubation in wässriger Lösung löst. Bei einem unlösbaren Haftkleber kann der Streifen der Vorrichtung nach der Analyse hingegen nicht entnommen und zur Dokumentation im getrockneten Zustand aufgehoben werden.

Als Alternativlösung offenbart die DE 20 2012 004 404 U1 die Möglichkeit, die Inkubationsrinne im Bodenbereich mit gegenüberliegenden Rastnasen zu versehen. Damit ist jedoch nur die Fixierung von relativ festen Teststreifen möglich. Die handelsüblichen weichen Teststreifen bieten nicht genug Widerstand, um das Einrasten von Rastnasen aus Kunststoffen zu ermöglichen. Es besteht weiterhin die Gefahr, dass die Streifen beim Einrasten beschädigt werden, wie in der DE 20 2012 004 404 U1 ausdrücklich erwähnt wird. Auch ist eine Entfernung aus der Inkubationsrinne nach dem Einrasten der Rastnasen umständlich, wenn nicht unmöglich.

Schließlich offenbart die DE 20 2012 004 404 U1 die Verwendung von Spangen zur Fixierung von Teststreifen in Bodennähe. Durch den Verschließmechanismus, in den die Streifen eingeklemmt werden können, besteht auch In dieser Variante die Gefahr von Beschädigungen Schließlich verdeckt eine Spange den Teststreifen in der Aufsicht wenigstens teilweise. Durch das Verdecken und durch Schattenbildung wird die Bilderfassung und anschließende automatische Diagnose erschwert.

Der vorliegenden Erfindung liegt die Aufgabe zu Grunde, eine verbesserte Inkubationsrinne bereitzustellen, die eine Inkubation von Teststreifen ermöglicht, wobei die Teststreifen so am Boden fixiert sind, dass sie bei der Inkubation in wässriger Lösung von allen Seiten bedeckt sind, auf der anderen Seite nach der Inkubation aber leicht entnommen werden können.

Gleichzeitig soll die Inkubationsrinne das Anfertigen hochqualitativer Bildaufnahmen der Streifen nach der Inkubation ermöglichen, die anschließend manuell oder automatisch ausgelesen und zur Diagnose ausgewertet werden können.

Die Fixierung der Teststreifen soll dabei derart erreicht werden, dass eine Beschädigung der Teststreifen, die häufig mit hochpreisigen, aufwändig herzustellenden analytischen Reagenzien, nahezu ausgeschlossen ist.

Diese und weitere Aufgaben werden durch den Gegenstand der vorliegenden Anmeldung und insbesondere auch durch den Gegenstand des beigefügten unabhängigen Anspruchs gelöst, wobei sich Ausführungsformen aus den Unteransprüchen ergeben.

Die der Erfindung zu Grunde liegende Aufgabe wird gelöst durch eine längliche Inkubationsrinne mit zwei einander gegenüber liegenden Längswänden, einer Vertiefung und einem Boden, bestückt mit einem länglichen Teststreifen mit einer Rückseite und einer Vorderseite, wobei der Teststreifen mit seiner Rückseite dem Boden der Inkubationsrinne zugewandt ist und auf seiner Vorderseite mit einem oder mehr als einem analytischen Reagenz beschichtet ist,
weiter umfassend wenigstens zwei Halteelemente zur Fixierung des Teststreifens, bevorzugt in Bodennähe,
dadurch gekennzeichnet, dass
die wenigstens zwei Halteelemente jeweils in Form eines aus der hervorstehenden Vorsprungs ausgebildet sind und, bevorzugt in Bodennähe, d.h. bevorzugte eine zur Seite und nach unten offene, eine, bevorzugt zur Fixierung des Halteelement in Bodennähe geeignete, Aussparung zur Aufnahme des Teststreifens aufweisen.

In einer bevorzugten Ausführungsform endet die Aussparung entlang der Querachse der Inkubationsrinne vor der Längswand.

In einer bevorzugten Ausführungsform reicht die Aussparung entlang der Querachse der Inkubationsrinne bis zur Längswand.

In einer bevorzugten Ausführungsform sind die wenigstens zwei Halteelemente in Paaren angeordnet.

In einer bevorzugten Ausführungsform weist die Inkubationsrinne wenigstens vier, bevorzugt wenigstens sechs, noch bevorzugter wenigstens acht in Paaren angeordnete Halteelemente auf.

In einer bevorzugten Ausführungsform sind die zwei Halteelemente eines Paares an den beiden Längsseiten einander gegenüber entlang der Längsachse der Inkubationsrinne diagonal versetzt angeordnet

In einer bevorzugten Ausführungsform sind die zwei Halteelemente eines Paares an den beiden Längsseiten auf der gleichen Höhe der Längsachse der Inkubationsrine entlang angeordnet.

In einer bevorzugten Ausführungsform ist der Vorsprung vertikal abgeschrägt.

In einer bevorzugten Ausführungsform ist der Vorsprung vertikal abgerundet.

In einer bevorzugten Ausführungsform ist der Vorsprung in Form eines Schafts ausgebildet.

In einer bevorzugten Ausführungsform verläuft die Längsachse des Vorsprungs senkrecht zur durch den Boden der Inkubationsrinne festgelegten Ebene.

In einer bevorzugten Ausführungsform verläuft die Längsachse des Vorsprungs senkrecht zur Längsachse der Inkubationsrinne.

In einer bevorzugten Ausführungsform verläuft der Vorsprung parallel zur Längsachse der Inkubationsrinne entlanggezogen.

In einer bevorzugten Ausführungsform sind die zwei Halteelemente an einem in die Vertiefung eingebrachten Rahmen angebracht, welcher bevorzugt auf wenigstens zwei Halterahmenfüßen auf dem Boden der Inkubationsrinne aufsitzt. Die Inkubationsrinne umfasst weiter den Teststreifen, der bevorzugt einen aus der Vertiefung ragenden Fortsatz zum Herausziehen aufweist.

Erfindungsgemäß kann die Inkubationsrinne ein Befestigungsmittel aufweisen, das geeignet ist, um die Inkubationsrinne mit wengistens einer weiteren Inkubationsrinne zu einem Verbund von Inkubationsrinnen zusammenzufügen. Im Verbund sind die Inkubationsrinnen als Einheit handhabbar. Sie können beispielsweise zusammen in ein Gerät eingesetzt, ausgegossen oder gewaschen werden, ohne dass dazu jede der Inkubationsrinnen im Verbund einzeln bewegt werden muss. Der Verbund umfasst wenigstens 2, bevorzugt wenigstens 3, 4, 5, 10, 12, 15, 20, 24, 36 oder 50 Inkubationsrinnen. Die Zahl der Inkubationsrinnen im Verbund ist im Wesentlichen durch die Aufnahmekapazität der Geräte begrenzt, in denen der Verbund und in den Einzelrinnen befindliche Proben prozessiert werden. Ebenso wird die Zahl der Inkubationsrinnen so begrenzt, dass der Verbund ausreichend stabil und nicht zu schwer wird. Bevorzugt besteht der Verbund aus im Wesentlichen oder tatsächlich identischen Inkubationsrinnen. Dabei kommt es jedoch lediglich auf die Berührungsflächen der Inkubationsrinnen an, die so gestaltet sein müssen, das das Befestigungsmittel und sonstige relevante Elemente wie der erfindungsgemäße Auflagerahmen - der einen Wand das Zusammenfügen mit der im Verbund Jeweils nächsten Inkubationsrinne gestattet. Sonstige Merkmale der einzelnen Inkubationsrinnen, beispielsweise Ihr Inhalt, ihre Kennzeichnung, ihre Breite o.ä. können variiert werden. Für analytische oder diagnostische Anwendungen kann jede der Inkubationsrinnen, auch wenn sie ansonsten gleich beschaffen oder gar identisch sind, eine Kennzeichnung aufweisen, die die darin zu untersuchende Probe einem Experiment bzw. Patienten zuordnet.

Das bevorzugte Befestigungsmittel weist ein vorstehendes Element und ein aufnehmendes Element (im Stand der Technik häufig als "männliches" bzw. "weibliches" Teil bezeichnet) auf, die ineinander gesteckt werden und einrasten, so dass die Verbindung zwischen den Inkubationsrinnen ausgebildet wird. Das vorstehende Element weist am oder nahe seinem Ende, das von der Inkubationsrinne wegzeigt, wenigstens eine Stelle auf, die breiter ist als das der Inkubationsrinnen zugewandte Ende. Das aufnehmende Element ist an diese Form angepasst, insbesondere dadurch, dass es nach oben zur Aufnahme des vorstehenden Elements und in Richtung der Außenseite der Längsrinne offen ist, so dass die beiden Elemente nach dem Einrasten nicht durch eine Bewegung in ausschließlich horizontaler Richtung voneinander getrennt werden können, sondern ein Herausheben oder -drehen mit einer vertikalen Komponente erforderlich ist, das vorstehende Element also von oben in das aufnehmende Element eingeführt wird, wobei die endgültige vertikale Position des vorstehenden Elements bevorzugt durch den Boden des aufnehmenden Elements bestimmt wird, auf den das vorstehende Element beim Einrasten aufsetzt. Beispielhafte Befestigungsmittel sind in verschiedenen Variationen im Stand der Technik offenbart, beispielsweise die als Schwalbenschwanz bezeichnete Verbindung.

Eine zum Verbund führende Verbindung zwischen Inkubationsnrinnen mit Befestigungsmitteln wie der klassischen Schwalbenschwanzverbindung kann man mehrfach einrichten und danach wieder lösen. In einer alternativen Ausführungsform weist die Inkubationsrinne ein Befestigungsmittel auf, mit dem die Inkubationswanne nur einmal mit einer weiteren Inkubationswanne zusammengefügt werden kann. Dies lässt sich zum Beispiel dadurch verwirklichen, dass im vorstehenden Element eine Sollbruchstelle eingefügt ist, während letzteres sehr fest und mit normalem Kraftaufwand nicht lösbar in das aufnehmende Element einrastet. Das Lösen der Verbindung ist dann nur unter Zerstörung des Befestigungsmittels möglich.

Eine besonders bevorzugte Variante des Befestigungsmittels ist die Schwalbenschwanzverbindung mit begradigter Kante. Dabei verläuft, von oben betrachtet, die dem Längsende der Inkubationsrinne zugewandte Kante des vorstehenden Elements senkrecht zur Längsachse der länglichen Inkubationsrinne. Der Vorteil besteht darin, dass die Wannen mit einer vertikaldrehenden Bewegung mit einander unter Ausbildung des Verbunds zusammengefügt bzw. wieder voneinander gelöst werden. Dies ermöglichst dem Anwender gegenüber der klassischen Schwalbenschwanzverbindung ein komfortableres Lösen von Inkubationsrinnen aus dem Verbund.

Bevorzugt weisen in dem erfindungsgemäßen Verbund sämtliche Inkubationsrinnen das gleiche Befestigungsmittel mit komplementären Elementen auf. Es ist jedoch auch möglich, dass verschiedene Inkubationsrinnentypen mit miteinander nicht oder nur in bestimmten Kombinationen kompatiblen Befestigungsmitteln im Verbund enthalten sind, beispielsweise um bestimmte Anordnungen oder Muster von verschiedenen Typen von Inkubationsrinnen innerhalb eines größeren Verbundes festzulegen, beispielsweise das Alternieren zweier Typen von Inkubationsrinnen. Beispielsweise kann Typ A einer Inkubationsrinne an der einen Seite das aufnehmende Element eines ersten Befestigungsmittels, z.B. einer Schwalbenschwanzverbindung, aufweisen und an der anderen Seite das vorstehende Element eines zweiten Befestigungsmittels, das z.B. einem Kugelgelenk nachgeahmt ist. Die Inkubationsrinne vom Typ A kann damit nicht direkt mit einer weiteren Inkubationsrinne vom Typ A zusammengefügt werden.

In einer bevorzugten Ausführungsform wird unter dem Begriff "Teststreifen", wie hierin verwendet, ein bevorzugt länglicher Träger verstanden, der gegen übliche Lösungsmittel, insbesondere auf wässriger Basis, chemisch ausreichend inert ist und mit einem Reagenz beschichtet ist, das für eine erwünschte chemische Reaktion, insbesondere ein analytisches Verfahren, besonders bevorzugt ein labordiagnostisches Verfahren, geeignet ist. Beispielsweise kann es sich um einen Nitrocellulosestreifen mit einem Antigen handeln. In einer bevorzugten Ausführungsform, wird unter dem Begriff "länglich", wie hierin verwendet, dass das Längenverhältnis der längeren zur kürzeren Seite in der Reihenfolge zunehmender Bevorzugung wenigstens 1,5:1, 2:1, 3:1, 4:1, 5:1, 7,5:1, 10:1, 15:1 oder 20:1 beträgt. In einer weiteren bevorzugten Ausführungsform wird unter dem Begriff "beschichtet", wie hierin verwendet, verstanden, dass das Reagenz derart mit dem Teststreifen verbunden ist, dass eine mit der Vorderseite des Streifens in Kontakt stehende Flüssigkeit auch mit Kontakt mit dem Reagenz tritt. Beispielsweise kann das Reagenz auf der Oberfläche der Vorderseite aufgeblottet sein, oder der Teststreifen kann an jeder Stelle eine im Wesentlichen einheitliche Konzentration des Reagenz aufweisen. Geeignete Teststreifen sind im Stand der Technik beschrieben und im Handel erhältlich, beispielsweise Linienblots von der Firma EUROIMMUN, Lübeck. In einer bevorzugten Ausführungsform wird unter dem Begriff "analytische Reagenz", wie hierin verwendet, eine chemische Verbindung verstanden, die mit einem in einer Probe oder sonstigen zu untersuchenden wässrigen Lösung enthaltenen Analyten chemisch oder physikalisch reagiert. Diese Reaktion kann nachgewiesen werden. Beispielsweise handelt es sich bei dem analytischen Reagenz um ein Antigen, besonders ein epitophaltiges Polypeptid, an das ein nachzuweisender Antikörper, bevorzugt Autoantikörper, aus einer zu untersuchenden Probe bindet, worauf der entstehende Antigen-Antikörper-Komplex mittels eines weiteren, enzymkonjugierten Antkörpers nachgewiesen werden kann. Alternativ kann es sich bei dem analytischen Reagenz z.B. um einen pH-abhängigen Farbstoff handeln, der eine bestimmte indikative Farbe annimmt, wenn er gegenüber einer Lösung mit einem bestimmten pH-Wert exponiert wird.

Ir einer bevorzugten Ausführungsform wird unter dem Begriff "Inkubationsrinne", wie hierin verwendet, ein bevorzugt flüssigkeitsdichter Behälter mit einem Boden, zwei Längswänden und zwei Querwänden verstanden. Die Inkubationswanne ist länglich ausgestaltet, um einen Teststreifen aufnehmen zu können. Bei einer Inkubationswanne von rechteckigem Umfang beträgt das Längenverhältnis von Querwand zu Längswand wenigstens 1:2, bevorzugter 1:3, 1:4, 1:5, 1:6, 1:7, 1:8, 1:9 oder 1:10. Bevorzugt besteht die Inkubationswanne aus einem gegenüber wässrigen Lösungen resistenten Material, beispielsweise Polystyrol, Polyethylen oder Polypropylen. In einer bevorzugten Ausführungsform Ist die Inkubationswanne mit einem Teststreifen bestückt und, optional, in einer Verpackung enthalten. In einer weiteren bevorzugten Ausführungsform enthält die Inkubationsrinne neben dem Teststreifen eine Flüssigkeit. Dabei kann es sich um eine zu untersuchende Probe, eine Waschlösung oder eine Lösung mit chemischen Reagenzien, Konservierungsmitteln oder Analyten handeln.

Die Inkubationsrinne kann rein manuell durch händisches Einbringen und Entfernen der benötigten Reagenzien für chemische Reaktionen, besonders analytische oder diagnostische Tests verwendet werden. Bevorzugt wird die Inkubationsrinne bzw, der Verbund aber in eine Vorrichtung eingebracht, die zahlreiche, idealerweise alle Schritte vollautomatisch ausführt, möglichst ohne dass die Anwesenheit von fachkundigem Personal erforderlich ist Eine solche Vorrichtung ist zur Bevorratung sowie zum Einbringen und Absaugen von geeigneten Puffern und Reagenzien ausgestattet, idealerweise zusätzlich zur Anfertigung geeigneter Bildaufnahmen vom voll prozessierten Teststreifen.

Die Inkubationsrinne ist bevorzugt konisch ausgestaltet, um die zur Bedeckung des Teststreifens notwendige Mindestvolumina an Flüssigkeit zu minimieren.

Bevorzugt weist die Inkubationsrinne einen Fortsatz an wenigstens einem, bevorzugt an den beiden Längsenden auf. Das hat den Vorteil, dass ein Schutz gegen das Verspritzen von Flüssigkeit in Richtung der Längsenden der Inkubationsrinne gegeben ist. Der Fortsatz, besonders sein Kopf, ist ein bevorzugter Ansatzpunkt für die Positionierung von Elementen des Befestigungsmittels. Eine Aushöhlung unter dem Verbindungsstück kann für die Auflage der Inkubationsrinne in eine Apparatur oder Transport- bzw. Inkubationswanne dienen, die eine entsprechend passende Erhöhung aufweist Die Inkubationsrinnen bzw. der Verbund ist dann innerhalb der Apparatur oder Wanne fixiert und kann nicht verrutschen, wenn die Inkubationsrinne bzw. der Verbund geschwenkt oder geschüttelt wird.

Optional kann die Inkubationsrinne bzw. der Verbund mit einer Abdeckung versehen werden. Dabei kann es sich um eine durchgehende Folie handeln, die den gesamten Verbund abdeckt, oder um einzelne, auf die Form der Inkubationsrinne abgestimmte Deckel.

Erfindungsgemäß kann die Inkubationsrinne weiterhin einen auf einer der Längswände angebrachten, bevorzugt waagerecht nach außen stehenden Auflagerahmen aufweisen. Dieser Auflagerahmen, der bevorzugt nicht die Vertiefung überdeckt, um die optische Betrachtung bzw. Bildaufnahme des Teststreifens nicht zu erschweren, kann eine beliebige Form annehmen, solange er auf die Längswand der weiteren Inkubationswand im Verbund so zugeschnitten ist, dass er im Verbund auf dieser aufliegt und damit eine in quer zur Längsachse der Inkubationsrinne lückenlose Abdeckung derart schafft, dass eine auf den Verbund auftropfende Flüssigkeit entweder auf den Rahmen oder die Vertiefung trifft Bevorzugt weist die Längswand, auf der der Auflagerahmen aufliegt, eine an ihn formangepasste Rahmenaussparung auf. Nicht als Teil des, aber analog zum Befestigungsmittel kann der Auflagerahmen dabei als vorstehendes Element und die Rahmenaussparung als aufnehmendes Element fungieren. Der Auflagerahmen steht bevorzugt in einem Winkel von 60 bis 120 °, bevorzugter 75 bis 105 °, noch bevorzugter 80 bis 100 °, am bevorzugtesten 90 °, d.h. waagerecht, von der senkrecht vom Boden abstehenden Längswand ab. Bei einer konischen Längswand wird der Winkel entsprechend einer an gleicher vertikaler Position mit dem Außenrahmen verbundenen senkrechten Längswand bemessen. Entscheidend ist, dass der Außenrahmen so gestaltet ist, dass er im Verbund auf der Längswand der weiteren Inkubationswand aufliegt.

Bevorzugt erstreckt sich der Auflagerahmen entlang der Längsachse der Inkubationsrinne wenigstens so weit wie die Vertiefung. Damit wird gewährleistet, dass seitlich verspritzte Flüssigkeit nicht zwischen den Inkubationsrinnen eines Verbundes durchläuft. Der Auflagerahmen ist bevorzugt parallel zum Boden der Inkubationsrinnen angeordnet. In einer bevorzugten Ausführungsform ist seine Unterseite derart ausgestaltet, dass er parallel auf der anderen Längswand der nächsten Inkubationrinne im Verbund aufliegt, seine Oberseite ist jedoch nach Art eines Pultdachs geneigt, so dass verspritzte Flüssigkeit in die Vertiefung zurückläuft.

Der Auflagerahmen bildet bevorzugt eine vertikale Verlängerung der Längswand, auf der er aufliegt Das bedeutet, dass er eine Verlängerung der Wand in dem Winkel bildet, indem die Wand sich vom Boden der Vertiefung erhebt, beispielsweise 90 ° bei einer senkrecht stehenden Wand und einem Winkel von mehr als 90 °C bei einer konisch ausgestalteten Inkubationsrinne, entsprechend dem Winkel, in dem die Längswand vom Boden der Vertiefung absteht.

Weiterhin kann die erfindungsgemäße Inkubationswanne ein System umfassend wenigstens zwei Halteelemente umfassen, die geeignet sind, den Teststreifen für die Durchführung von chemischen und biologischen Reaktionen, insbesondere labordiagnostischen Verfahren, in geeigneter Weise in der Inkubationsrinne zu positionieren. Dazu gehört insbesondere, dass ein Aufschwimmen, welche zur Austrocknung und Abkehr wenigstens einer Seite des Teststreifens von reagenzhaltigen Flüssigkeiten führt, sowie das Umdrehen des Teststreifens, welches die Betrachtung oder Bildaufnahme stört, verhindert werden muss.

Erfindungsgemäß wird die Aufgabe durch Halteelemente in der Inkubationsrinne gelöst, die aus wenigstens einer Längswand hervortreten, wobei bevorzugt ist, dass die beiden Längswände der Inkubationsrinne jeweils wenigstens ein Halteelement aufweisen. Jedes Halteelement besteht aus einem in Bodennähe aus der Längswand hervorstehenden Vorsprung. Dieser ragt so weit ins Innere der Vertiefung, dass er, insbesondere in Kombination mit einem weiteren Halteelement, bevorzugt an der gegenüberliegenden Längswand, geeignet ist, die Bewegung eines darunter eingeführten Teststreifens in vertikaler Richtung insoweit zu begrenzen. Bei der Durchführung des Tests kann dann so viel Flüssigkeit in die Inkubationsrinne gegeben werden, dass der Streifen in ausreichendem Maße bedeckt ist Die Menge der Flüssigkeit kann durch geeignete Anordnungen minimiert werden, die ein Schwenken der Inkubationsrinne gestatten, wobei die Flüssigkeit von einem zum anderen Längsende zum anderen und wieder zurück fließt. Derartige Anordnungen sind im Stand der Technik beschrieben, beispielsweise in der EP 08169 465.5 oder EP 2191893.

Unterhalb des Vorsprungs weist das Halteelement eine Aussparung auf, die so groß ist, dass der Teststreifen darunter zwar Platz findet und bevorzugt bei einer Bewegung der Inkubationsrinne zur optimalen Exponierung gegenüber der umgebenden Flüssigkeit und darin enthaltener Reagenzien leichte Aufwärts- und Abwärtsbewegungen ausführen, sich aber nicht um seine Längsachse drehen kann. Je nach Breite des Streifens und der Inkubationsrinne kann die Aussparung entlang der Querachse der Inkubationsrinne vor der Längswand enden. Im Gegensatz dazu ist es auch möglich, dass die Aussparung entlang der Querachse der Inkubationsrinne bis zur Längswand reicht, d.h. sie umfasst die gesamte Strecke zwischen der Längswand, aus der der Vorsprung herausragt, und dem Ende des Vorsprungs im Inneren der Inkubationsrinne. Der Teststreifen kann sich für den Fall, dass er auf beiden Seiten durch derartig beschaffene Halteelemente positioniert wird, über die gesamte Breite der Inkubationsrinne bewegen, ist jedoch durch die Vorsprünge an einem Aufschwimmen bzw. Drehen gehindert.

Mit Hinblick auf die Länge des Vorsprungs eines Halteelements ist es essentiell, dass dieser keinesfalls bis zur gegenüberliegenden Längsseite reicht. Bevorzugt entspricht die Länge des Vorsprungs über der Querachse der Inkubationsrinne am Boden weniger als 75, noch bevorzugter weniger als 50, 45, 40, 30, 25 oder 20 % der Breite der Inkubationsrinne am Boden. In einer weiteren bevorzugten Ausführungsform ist ein in der Mitte der Inkubationsrinne liegender Teststreifen, dessen Breite 25, bevorzugter 50, noch bevorzugter 70 % der Breite der Inkubationswanne aufweist, über höchstens 30 % seiner Breite durch den Vorsprung des Halteelements überdeckt.

Die erfindungsgemäßen Halteelemente positionieren den Teststreifen besonders effektiv, wenn sie in Paaren angeordnet sind. Das ist bei zwei Halteelementen dann der Fall, wenn sie entlang der Längsachse der Inkubationsrinne auf gegenüberliegenden Längsachsen relativ zu den anderen Halteelementen so angeordnet sind, dass ihre Zusammengehörigkeit aufgrund ihrer räumlichen Nähe erkennbar ist. Dabei können die zwei Halteelemente eines Paares an den beiden Längsseiten einander gegenüber entlang der Längsachse der Inkubationsrinne diagonal versetzt oder auf der gleichen Höhe der Längsachse der Inkubationsrine entlang angeordnet sein. Bevorzugt beträgt der Abstand der beiden Halteelemente eines Paares entlang der Langsachse der Inkubationsrinne höchsten 40, bevorzugter, 30, bevorzugter, 20, bevorzugter 20, bevorzugter 10, bevorzugter 7,5, bevorzugter 5, bevorzugter 2,5 % der Länge der Vertiefung der Inkubationsrinne. Die Halteelemente können auch alternierend entlang der Längsachse auf beiden Seiten der Inkubationswanne angeordnet sein.

Das Halteelement kann die Form eines horizontal gespiegelten Buchstabens L annehmen. Besonders bevorzugt ist das Halteelement vertikal abgeschrägt, abgerundet oder in Form eines Schafts ausgeprägt. Auf diese Weise ist die schattenbildende Fläche verringert. Die verringerte Schattenbildung auf dem Teststreifen sorgt für eine bessere optische Zugänglichkeit, die eine verbesserte Erkennbarkeit bzw. Bildqualität von gewonnnenen Aufnahmen bedingt. Die Ausbildung mit einer vertikal abgeschrägten oder abgerundeten Form stellt einen besonders vorteilhaften Kompromiss zwischen Stabilität des Halteelementes und Minimierung der Schattenbildung dar. Die Halteelemente sind geeignet, um den Teststreifen in Bodennähe zu fixieren, worunter In einer bevorzugten Ausführungsform eine Position einem vertikalen Bereich verstanden wird, der die untersten 80, bevorzugter 60, bevorzugter 50, bevorzugter 40, bevorzugter 30, bevorzugter 20, bevorzugter 15, bevorzugter 10, bevorzugter 5 % der Höhe der Vertiefung der Inkubationsrinne umfasst.

Bevorzugt sind Inkubationsrinne und die Halteelemente in einem Stück gefertigt. Die Halteelemente stehen dann direkt aus den Längswänden der Inkubationsrinne hervor. Alternativ können die Halteelemente in Form eines Halteelementrahmens separat von der Inkubationsrinne bereitgestellt werden. Der Halteelementrahmen kann durch einen oder mehr als einen, bevorzugt mindestens zwei Halteelementrahmenfüße auf dem Boden der Inkubationsrinne aufgesetzt werden, wobei die Höhe der Halteelementrahmenfüße den Abstand des Halteelementrahmens vom Boden der Inkubationswanne bestimmt. Alternativ kann auch die Form bzw. der Umfang des Halteelementrahmens so angepasst werden, dass er bei einer konischen Inkubationsrinne direkt auf den Längswänden aufsetzt. Dazu ist der Halteelementrahmen enger als die Vertiefung nahe dem oberen Ende der Längswände, aber breiter als am Boden der Inkubationsrinne zu gestalten. Bevorzugt ist der Halteelementrahmen so beschaffen, dass er auf einem Niveau von weniger als 80, bevorzugter 60, bevorzugter 50, bevorzugter 40, bevorzugter 30, bevorzugter 20 oder bevorzugter 15 % der Höhe der Vertiefung der Inkubationsrinne positioniert ist. Sofern das Eigengewicht des Halteelementrahmens nicht genügt, kann er durch zusätzliche Gewichte beschwert werden.

Ein erfindungsgemäßes Verfahren kann einen oder mehr als einen der folgenden Schritte umfassen, bevorzugt, aber nicht notwendigerweise in der genannten Reihenfolge:
a) Bereitstellen der erfindungsgemaßen Inkubationsrinne umfassend den Teststreifen,
b) Einbringen von Probenmaterial in einer Flüssigkeit, bevorzugt wässrigen Lösung, in die Blotwanne, so dass dieses in Kontakt mit dem Teststreifen tritt,
c) Inkubation des Teststreifens in Anwesenheit des Probenmaterials,
d) Entfernung des Probenmaterials,
e) Waschen des Teststreifens
f) Einbringen von wenigstens einem chemischen Reagenz und Inkubation mit dem Teststreifen aus Schritt d),
g) Optional wenigstens einmalige Wiederholung von Schritt f) mit dem gleichen oder einem anderen chemischen Reagenz
h) Optional Waschen des Teststreifens
i) Optional Entnahme des Teststreifens
j) Optional Analyse des Teststreifens .
k) Optional Trocknen und ggf. Archivieren des Teststreifens.

Im Folgenden wird die Erfindung unter Bezugnahme auf die Figuren anhand von Ausführungsbeispielen erläutert. Die beschriebenen Ausführungsformen sind in jeder Hinsicht lediglich beispielhaft und nicht als einschränkend zu verstehen, und verschiedene Kombinationen der angeführten Merkmale sind vom Umfang der Erfindung umfasst.

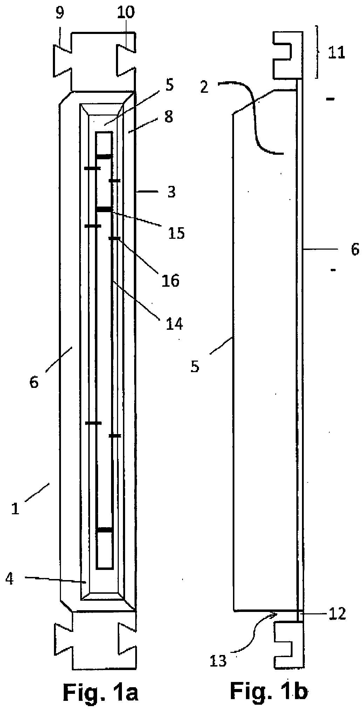

**Fig. 1a** zeigt eine von oben betrachtete erfindungsgemäße Inkubationsrinne (1) mit Längswänden ((3) und (2), letztere nicht sichtbar unter dem Auflagerahmen (6)) und einer Vertiefung (4), in der sich auf dem Boden (5) ein Teststreifen (14) mit Reagenz (15) befindet, der durch Halteelemente (16) fixiert ist. Die eine Längsseite weist einen Auflagerahmen (6) auf, die andere einen an einen passenden Auflagerahmen Rahmenaussparung (8). Dargestellt ist außerdem das vorstehende (9) und aufnehmende (10) Element des Befestigungsmittels.
   Wie in **Fig. 1b** von der Seite gezeigt, befindet sich an den Längsenden jeweils ein Kopf (11) mit einem vorstehenden (9) und einem aufnehmenden Element (10). Der Kopf ist über ein Verbindungsstück (12), das darunter eine Aushöhlung (13) aufweist, mit den übrigen Teilen der Inkubationswanne verbunden.

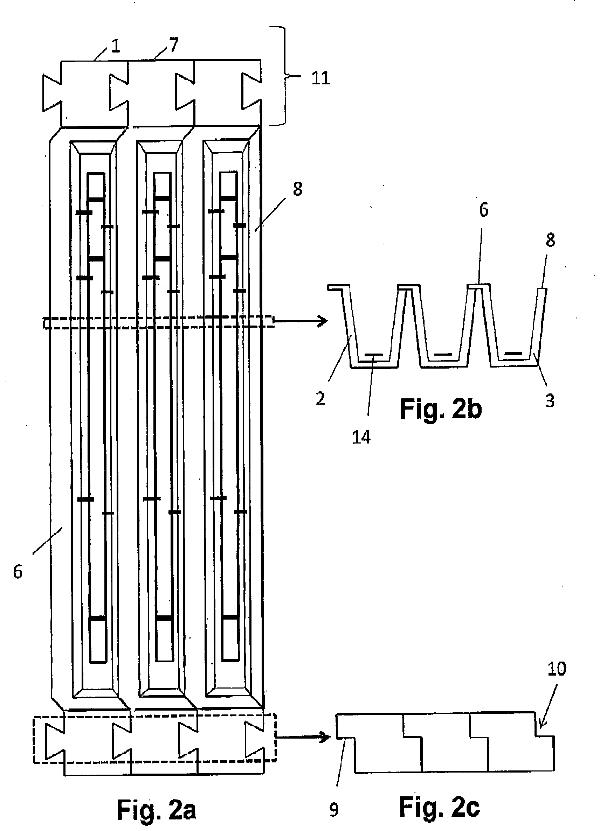

**Fig. 2a** zeigt einen erfindungsgemäßen Verbund von drei identischen Inkubationswannen, wie sie in **Fig. 1a** gezeigt sind. Der Verbund wird fixiert durch den in die Rahmenaussparung (8) passenden Auflagerahmen (6) sowie durch das in das aufnehmende Element (10) gesteckte und eingerastete vorstehende Element (9).
**Fig. 2b** illustriert im Querschnitt, wie der Auflagerahmen (6) so an die Rahmenaussparung (8) der nächstliegenden Inkubationswanne im Verbund angepasst ist, dass der Zwischenraum gegen ein mögliches Verspritzen von Flüssigkeit abgeschlossen und abgedichtet ist, besonders bei konisch geformten Inkubationsrinnen.
**Fig. 2c** illustriert im Querschnitt, wie vorstehendes Element (9) und aufnehmendes Element (10) im Verbund eingerastet sind.

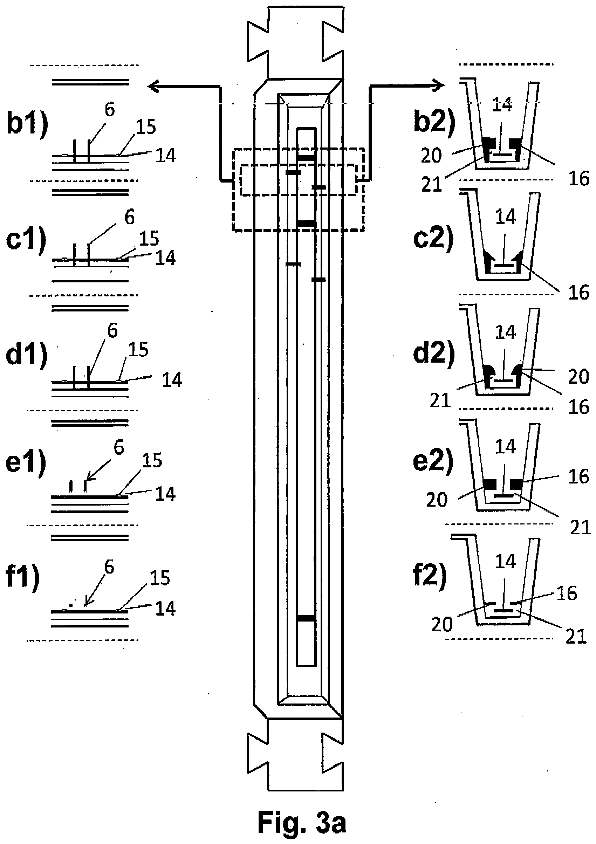

**Fig. 3** zeigt verschiedene erfindungsgemäße Ausgestaltungen des Halteelemente (6) ausgehend von der **Fig. 1a** entsprechenden Inkubationsrinne in **Fig. 3a****.** Kennzeichnend für alle erfindungsgemäßen Halteelemente ist der aus der oder aus Richtung der Längswand herausragende Vorsprung (20), unter dem sich eine Aussparung (21) befindet. Der Vorsprung begrenzt in vertikaler Richtung die Lage eines ein unter ihm eingeführten Teststreifens (14) mit einem Reagenz (15) durch ein Paar ausbildende Halteelemente auf gegenüberliegenden Längsseiten der Inkubationsrinne. Der Teststreifen wird auf diese Weise in einer Maximalhöhe am Boden gehalten wird, die der unteren Kante des Vorsprungs entspricht. Seine Lage in horizontaler Richtung beschränkt sich durch die Form der Aussparung (21) der Halteelemente.
   Die **Figs. 3b1), 3c1), 3d1), 3e1)** und **3f1)** bzw. **3b2), 3c2), 3d2), 3e2)** und **3f2)** zeigen im Querschnitt entlang der Längsachse bzw. Querachse der Inkubationsrinne verschiedene Abwandlungen des Halteelementes, insbesondere mit vertikal abgeschrägtem Vorsprung **(3c1)** und **3c2)),** vertikal abgerundetem Vorsprung **(3d1)** und **3d2)**), Halteelemente, bei denen die Aussparung entlang der Querachse der Inkubationsrinne vor der Längswand endet **(3b2), 3c2)** und **3d2))** und, im Gegensatz zu den letzteren, Halteelemente, bei den denen die Aussparung bis zur Längswand reicht **(3e2)** und **3f2)**)**.** Im letzteren Fall ist die Lage des Teststreifens in horizontaler Richtung ausschließlich durch die beiden Längswände begrenzt. Die **Figs. 3f1)** und **3f2)** zeigen in Form von Schaften ausgebildete Halteelemente.

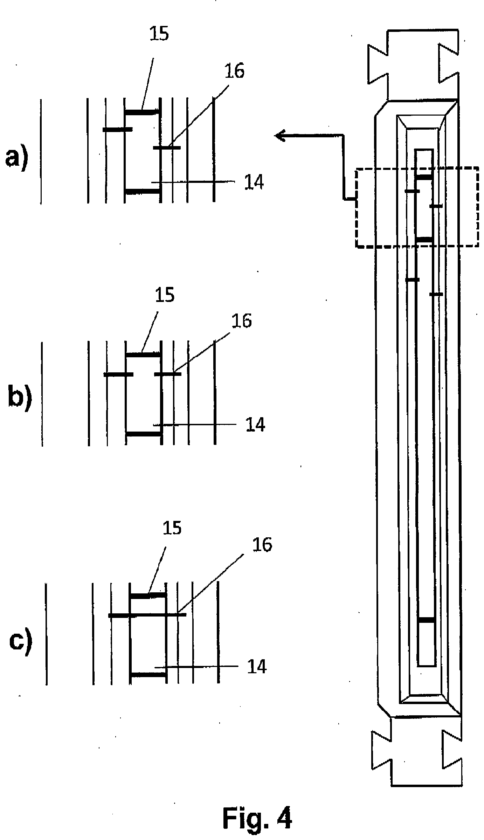

**Fig. 4** zeigt verschiedene Möglichkeiten zur Anordnung von zwei Halteelementen an gegenüberliegenden Längsseiten in Paaren anhand einer von oben betrachteten Inkubationsrinne, wobei die **Figs. 4a), 4b)** und **4c****)** die in **Figs. 1a** und **3a** dargestellte Variante in Vergrößerung (**Fig. 4a**)) mit zwei möglichen Abwandlungen **(4b)** und **4c)**) illustrieren. In **Fig. 4a****)** sind die zwei Halteelemente des Paares an den beiden Längsseiten auf der gleichen Höhe der Längsachse der Inkubationsrine entlang angeordnet. In **Fig. 4b****)** sind die zwei Halteelemente des Paares an den beiden Längsseiten einander gegenüber entlang der Längsachse der Inkubationsrinne diagonal versetzt angeordnet. Leglich Illustrativ zum Vergleich zeigt **Fig. 4c****)** eine nicht erfindungsgemäße Abwandlung, in der die zwei Halteelemente an den beiden Längsseiten auf der gleichen Höhe der Längsachse der Inkubationsrine entlang angeordnet und in Form einer Spange verbunden sind, so dass sie den Teststreifen überdecken.

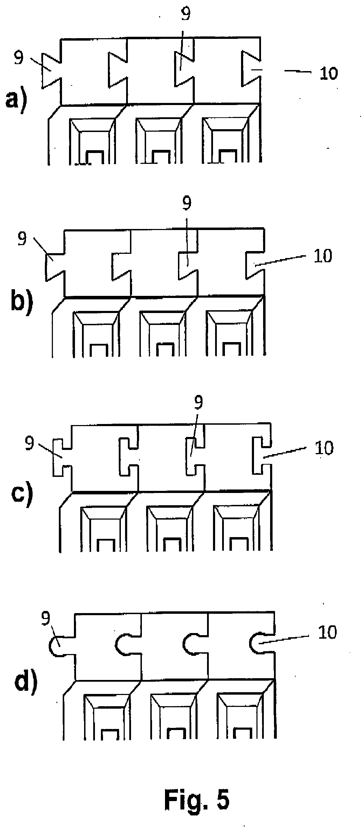

**Fig. 5** zeigt verschiedene Möglichkeiten zur Ausgestaltung des Befestigungsmittels mit einem vorstehenden Element (9) und einem aufnehmenden Element (10) **Fig. 5a****)** zeigt die sogenannte Schwalbenschwanzverbindung. **Fig. 5b****)** zeigt die Schwalbenschwanzverbindung mit einer begradigten Kante **Flg. 5c)** zeigt ein Befestigungsmittel mit stempelförmigem vorstehenden Element. **Fig. 5d**) zeigt ein Befestigungsmittel, bei dem das vorstehende Element kreisförmig bzw. dreidimensional in Form eines Kugelgelenks ausgestaltet ist.

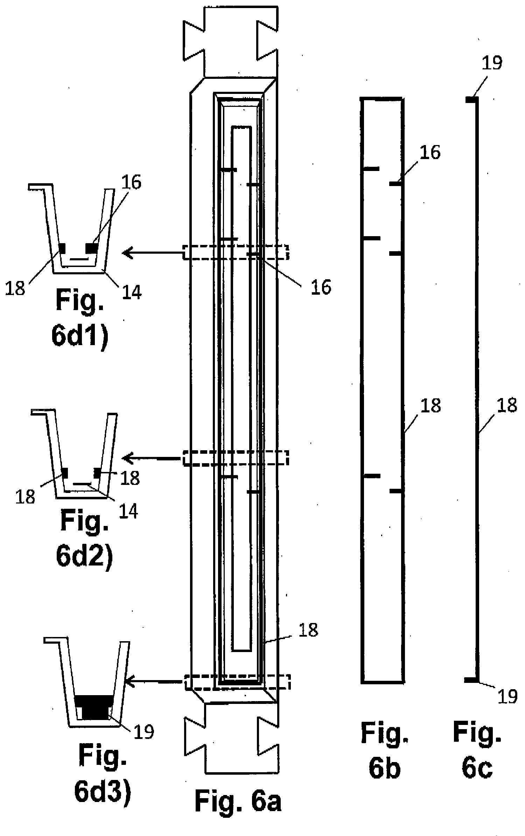

**Fig. 6** verdeutlicht die Möglichkeit, die Halteelemente in Form eines Halteelementrahmens (18) in die Inkubationsrinne einzubringen, der optional auf einem oder mehr als einem Halterahmenelementfuß (19) steht. Gezeigt sind von oben Inkubationsrinne samt Halteelementrahmen **(****Fig. 6a**)), der Halteelementrahmen allein in gleicher Ansicht **(****Fig. 6b**)), der Halteelementrahmen im Querschnitt entlang der Längsachse der Inkubationsrinne **(****Fig. 6c**)) sowie verschiedene Querschnitte des Halterahmens in der Inkubationsrinne, genauer an einer Stelle mit Halteelement **(****Fig. 6d1)**), ohne Halteelement **(****Fig. 6d2)**) und am Längsende durch den Halteelementfuß **(****Fig. 6d3**)).

### Bezugszeichenliste

1 Längliche Inkubationsrinnen
2 und 3 Längswände
4 Vertiefung
5 Boden
6 Auflagerahmen
7 weitere Inkubationsrinnen
8 Rahmenaussparung
9 vorstehendes Element
10 aufnehmendes Element
11 Kopf
12 Verbindungsstück
13 Aushöhlung
14 Teststreifen
15 Reagenz
16 Halteelement
17 Verbund
18 Halteelementrahmen
19 Halteelementrahmenfuß
20 Vorsprung
21 Aussparung

## Patentansprüche

1. Längliche Inkubationsrinne mit zwei einander gegenüber liegenden Längswänden, einer Vertiefung und einem Boden,
bestückt mit einem länglichen Teststreifen mit einer Rückseite und einer Vorderseite,
wobei der Teststreifen mit seiner Rückseite dem Boden der Inkubationsrinne zugewandt ist und auf seiner Vorderseite mit einem oder mehr als einem analytischen Reagenz beschichtet ist,
weiter umfassend wenigstens zwei Halteelemente zur Fixierung des Teststreifens,
**dadurch gekennzeichnet, dass**
die wenigstens zwei Halteelemente jeweils in Form eines aus wenigstens einer Längswand hervorstehenden Vorsprungs ausgebildet sind und eine Aussparung zur Aufnahme des Teststreifens aufweisen, wobei die Aussparung so groß ist, dass der Teststreifen darunter zwar Platz findet und bevorzugt bei einer Bewegung der Inkubationsrinne zur optimalen Exponierung gegenüber der umgebenden Flüssigkeit und darin enthaltener Reagenzien leichte Aufwärts- und Abwärtsbewegungen ausführen, sich aber nicht um seine Längsachse drehen kann.

2. Inkubationsrinne nach Anspruch 1, wobei die Aussparung entlang der Querachse der Inkubationsrinne vor der Längswand endet.

3. Inkubationsrinne nach Anspruch 1, wobei die Aussparung entlang der Querachse der Inkubationsrinne bis zur Längswand reicht.

4. Inkubationsrinne nach einem der Ansprüche 1 bis 3, wobei die wenigstens zwei Halteelemente in Paaren angeordnet sind.

5. Inkubationsrinne nach Anspruch 4, aufweisend wenigstens vier, bevorzugt wenigstens sechs, noch bevorzugter wenigstens acht in Paaren angeordnete Halteelemente.

6. Inkubationsrinne nach einem der Ansprüche 4 bis 5, wobei die zwei Halteelemente eines Paares an den beiden Längsseiten einander gegenüber diagonal versetzt angeordnet sind.

7. Inkubationsrinne nach einem der Ansprüche 4 bis 6, wobei die zwei Halteelemente eines Paares an den beiden Längsseiten auf der gleichen Höhe der Längsachse der Inkubationsrinne entlang angeordnet sind.

8. Inkubationsrinne nach einem der Ansprüche 1 bis 7, wobei der Vorsprung vertikal abgeschrägt ist.

9. Inkubationsrinne nach einem der Ansprüche 1 bis 7, wobei der Vorsprung vertikal abgerundet ist.

10. Inkubationsrinne nach einem der Ansprüche 1 bis 7, wobei der Vorsprung in Form eines Schafts ausgebildet ist.

11. Inkubationsrinne nach einem der Ansprüche 1 bis 10, wobei die Längsachse des Vorsprungs senkrecht zur durch den Boden der Inkubationsrinne festgelegten Ebene verläuft.

12. Inkubationsrinne nach einem der Ansprüche 1 bis 11, wobei die Längsachse des Vorsprungs senkrecht zur Längsachse der Inkubationsrinne verläuft.

13. Inkubationsrinne nach einem der Ansprüche 1 bis 10, wobei der Vorsprung parallel zur Längsachse der Inkubationsrinne entlanggezogen verläuft.

14. Inkubationsrinne nach einem der Ansprüche 1 bis 13, wobei die zwei Halteelemente an einem in die Vertiefung eingebrachten Rahmen angebracht sind, welcher bevorzugt auf wenigstens zwei Halterahmenfüßen auf dem Boden der Inkubationsrinne aufsitzt.

15. Inkubationsrinne nach einem der Ansprüche 1 bis 14, wobei der Teststreifen einen aus der Vertiefung ragenden Fortsatz zum Herausziehen aufweist.

## Claims

1. An elongated incubation channel with two opposite longitudinal walls, a recess, and a base,
equipped with an elongated test strip having a rear surface and a front surface,
wherein the test strip faces the base of the incubation channel with its rear surface and is coated on its front surface with one or more analytical reagents,
further comprising at least two holding elements for fixation of the test strip, **characterized in that**
the at least two holding elements are each formed by a respective projecting element extending out from at least one of the longitudinal walls and furthermore having a notch for receiving the test strip, wherein the notch is of a size such that the test strip fits below it and preferably can perform slight upward and downward movements during a movement of the incubation channel for optimal exposition against the surrounding liquid and the reagents located therein, but wherein the test strip cannot turn around its longitudinal axis.

2. The incubation channel according to claim 1, wherein the notch ends along the transverse axis of the incubation channel before the longitudinal wall.

3. The incubation channel according to claim 1, wherein the notch extends along the transverse axis of the incubation channel up to the longitudinal wall.

4. The incubation channel according to any of claims 1 to 3, wherein the at least two holding elements are arranged in pairs.

5. The incubation channel according to claim 4, wherein the incubation channel has at least four, preferably at least six, and even more preferably at least eight holding elements arranged in pairs.

6. The incubation channel according to any of claims 4 to 5, wherein the two holding elements of a pair are arranged on the two longitudinal sides and are furthermore diagonally offset with respect to each other.

7. The incubation channel according to any of claims 4 to 6, wherein the two holding elements of a pair are arranged on the two longitudinal sides at a same height along the longitudinal axis of the incubation channel.

8. The incubation channel according to any of claims 1 to 7, wherein the projecting element is vertically bevelled.

9. The incubation channel according to any of claims 1 to 7, wherein the projecting element is vertically rounded off.

10. The incubation channel according to any of claims 1 to 7, wherein the projecting element is configured in the form of a shaft.

11. The incubation channel according to any of claims 1 to 10, wherein the longitudinal axis of the projecting element runs perpendicular to the level determined by the base of the incubation channel.

12. The incubation channel according to any of claims 1 to 11, wherein the longitudinal axis of the projecting element runs perpendicular to the longitudinal axis of the incubation channel.

13. The incubation channel according to any of claims 1 to 10, wherein the projecting element runs parallel to the longitudinal axis of the incubation channel.

14. The incubation channel according to any of claims 1 to 13, wherein the two holding elements are positioned in a frame placed in the recess, wherein the frame preferably rests on at least two feet of the holding element on the base of the incubation channel.

15. The incubation channel according to any of claims 1 to 14, wherein the test strip comprises an appendage protruding from the recess for extraction.

## Revendications

1. Canal d'incubation allongé avec deux parois longitudinales placées l'une en face de l'autre, un creux et un fond, garni d'une bande-test allongée avec un côté arrière et un côté avant,
dans lequel la bande-test est tournée avec son côté arrière vers le fond du canal d'incubation et est revêtue sur son côté avant d'un ou de plus d'un réactif analytique,
comprenant en outre au moins deux éléments de maintien pour la fixation de la bande-test,
**caractérisé en ce que** lesdits au moins deux éléments de maintien sont réalisés respectivement sous la forme d'une protubérance saillante sur au moins une paroi longitudinale et présentent une découpe destinée à recevoir la bande-test, dans lequel la découpe est d'une grandeur telle que la bande-test trouve place en dessous de celle-ci et puisse de préférence effectuer de légers mouvements vers l'amont et l'aval lors d'un mouvement du canal d'incubation en vue d'une exposition optimale par rapport au liquide qui l'entoure et aux réactifs contenus dans celui-ci, mais qu'elle ne puisse pas tourner autour de son axe longitudinal.

2. Canal d'incubation selon la revendication 1, dans lequel la découpe se termine avant la paroi longitudinale le long de l'axe transversal du canal d'incubation.

3. Canal d'incubation selon la revendication 1, dans lequel la découpe s'étend jusqu'à la paroi longitudinale le long de l'axe transversal du canal d'incubation.

4. Canal d'incubation selon l'une quelconque des revendications 1 à 3, dans lequel lesdits au moins deux éléments de maintien sont disposés par paires.

5. Canal d'incubation selon la revendication 4, présentant au moins quatre, de préférence au moins six, de préférence encore au moins huit éléments de maintien disposés par paires.

6. Canal d'incubation selon l'une quelconque des revendications 4 à 5, dans lequel les deux éléments de maintien d'une paire sont disposés en décalage diagonal l'un par rapport à l'autre sur les deux côtés longitudinaux.

7. Canal d'incubation selon l'une quelconque des revendications 4 à 6, dans lequel les deux éléments de maintien d'une paire sont disposés sur les deux côtés longitudinaux à la même hauteur le long de l'axe longitudinal du canal d'incubation.

8. Canal d'incubation selon l'une quelconque des revendications 1 à 7, dans lequel la protubérance est chanfreinée verticalement.

9. Canal d'incubation selon l'une quelconque des revendications 1 à 7, dans lequel la protubérance est arrondie verticalement.

10. Canal d'incubation selon l'une quelconque des revendications 1 à 7, dans lequel la protubérance est réalisée à la forme d'une tige.

11. Canal d'incubation selon l'une quelconque des revendications 1 à 10, dans lequel l'axe longitudinal de la protubérance est perpendiculaire au plan défini par le fond du canal d'incubation.

12. Canal d'incubation selon l'une quelconque des revendications 1 à 11, dans lequel l'axe longitudinal de la protubérance est perpendiculaire à l'axe longitudinal du canal d'incubation.

13. Canal d'incubation selon l'une quelconque des revendications 1 à 10, dans lequel la protubérance est étendue parallèlement à l'axe longitudinal du canal d'incubation.

14. Canal d'incubation selon l'une quelconque des revendications 1 à 13, dans lequel les deux éléments de maintien sont montés sur un cadre introduit dans le creux, qui repose de préférence sur au moins deux pieds de cadre de maintien sur le fond du canal d'incubation.

15. Canal d'incubation selon l'une quelconque des revendications 1 à 14, dans lequel la bande-test présente un prolongement s'étendant hors du creux en vue de son extraction.
